(19) Europäisches Patentamt
European Patent Office
Office européen des brevets

(11) Veröffentlichungsnummer : **0 318 490 B1**

(12) # EUROPÄISCHE PATENTSCHRIFT

(45) Veröffentlichungstag der Patentschrift :
**29.01.92 Patentblatt 92/05**

(51) Int. Cl.$^5$ : **C07J 53/00,** A61K 31/565

(21) Anmeldenummer : **87905178.7**

(22) Anmeldetag : **11.08.87**

(86) Internationale Anmeldenummer :
**PCT/DE87/00361**

(87) Internationale Veröffentlichungsnummer :
**WO 88/01275 25.02.88 Gazette 88/05**

(54) **14,17-g(b)-ETHANO-14-g(b)-ESTRATRIENE, VERFAHREN ZU DEREN HERSTELLUNG UND DIESE ENTHALTENDE PHARMAZEUTISCHE PRÄPARATE.**

(30) Priorität : **20.08.86 DE 3628189**

(43) Veröffentlichungstag der Anmeldung :
**07.06.89 Patentblatt 89/23**

(45) Bekanntmachung des Hinweises auf die Patenterteilung :
**29.01.92 Patentblatt 92/05**

(84) Benannte Vertragsstaaten :
**AT BE CH DE FR GB IT LI LU NL SE**

(56) Entgegenhaltungen :
**Chemical Abstracts, Vol. 105, Nr. 17 27 October 1986, (Columbus, Ohio, US), J.R. Bull et al.: "Cycloaddition route to 14a-formylestrone and derived 14a-substituted products", see page 724, abstract 153382n, & J. Chem. Soc. Chem.Comm. 1986, (6), 451-3**
**Journal of Medicinal Chemistry, Vol. 16, Nr. 3 March 1973, (Washington, DC, US) A.J. Solo et al.:"Ring D bridged steroid analogs. 11. The high Clauberg activity of 19-Nor-14a, 17a-ethano-4-pregnene-3, 20-dione", see pages 270-273**

(73) Patentinhaber : **SCHERING AKTIENGESELLSCHAFT Berlin und Bergkamen**
**Müllerstrasse 170/178 Postfach 65 03 11**
**W-1000 Berlin 65 (DE)**

(72) Erfinder : **BULL, James, R.**
**342 Ridgeview Road Waterkloff Ridge 2**
**Pretoria 0181 (ZA)**
Erfinder : **THOMSON, Russel, I.**
**478 Grunberger Street Constantia Park**
**Pretoria (ZA)**
Erfinder : **LAURENT, Henry**
**Glambecker Weg 21**
**W-1000 Berlin 28 (DE)**
Erfinder : **SCHRÖDER, Helmut**
**Wartenburgstrasse 16**
**W-1000 Berlin 61 (DE)**
Erfinder : **WIECHERT, Rudolf**
**Petzower Strasse 8a**
**W-1000 Berlin 39 (DE)**

EP 0 318 490 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft neue östrogen wirksame Mittel, enthaltend eine Verbindung der allgemeinen Formel I

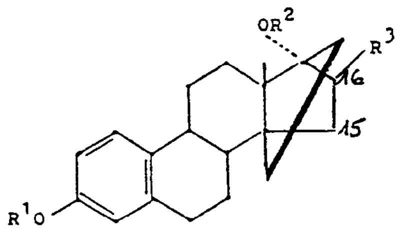

(I),

worin
$R^1$ ein Wasserstoffatom, eine Methyl- oder eine Acylgruppe mit 1 - 12 Kohlenstoffatomen,
$R^2$ ein Wasserstoffatom oder eine Acylgruppe mit 1 - 12 Kohlenstoffatomen,
$R^3$ ein Wasserstoffatom oder eine Methylgruppe
bedeuten.

Als Acylgruppen $R^1$ und $R^2$ kommen Reste von organischen Carbonsäuren mit 1 - 12 Kohlenstoffatomen infrage. Sie leiten sich ab von aliphatischen, cycloaliphatischen, aliphatisch-cycloaliphatischen, cycloaliphatisch-aliphatischen und aromatischen Monocarbonsäuren. Die Anzahl der Kohlenstoffatome im Ring variiert von 3 bis 7. Bevorzugt werden als Reste $R^1$ und $R^2$, die Acylgruppen der Essig-, Propion-, Butter-, Isobutter-, Pivalin-, Capron-, Acryl-, Croton-, Heptyl-, Capryl-, Pelargon-, Decan-, Undecan-, Dodecan-, 3-Cyclopentylpropion- und Benzoesäure enthalten.

Verbindungen der allgemeinen Formel I mit einer ungesättigten 15, 16-Kohlenstoffdoppelbindung, $R_1$ in der Bedeutung von Wasserstoff, Methyl und Acetyl, $R_2$ in der Bedeutung von Wasserstoff und Acetyl und $R_3$ in der Bedeutung von Wasserstoff, werden in J. Chem. Soc., Chem. Commun. 1986, 451-453, als Zwischenprodukte zur Herstellung von 14α-Formylestron und anderer 14α-substituierter Verbindungen beschrieben.

Es wurde nun gefunden, daß die in J. Chem. Soc. beschriebenen $\Delta^{15}$-Verbindungen und die noch nicht beschriebenen entsprechenden 15,16-Dihydroverbindungen im Allen-Doisy-Test auf östrogene Wirkung nach sub utaner und nach peroraler Applikation stärker östrogen wirksam sind als Ethinylestradiol.

Im Allen-Doisy-Test nimmt man eine Bewertung von Vaginalabstrichen bei ovariektomierten Ratten an den Taben 3, 4, 5 und 8 nach der einmaligen Applikation der Prüfsubstanz vor. Folgende Zyklusstadien werden unterschieden:

1 = Diöstrus (Leukozyten und kernhaltige Epithelzellen),
2 = Proöstrus (kernhaltige Ephitelzellen),
3 = östrus (kernlose Hornschollen),
4 = Metöstrus (kernlose Hornschollen, Leukozyten, Epithelzellen)

östrogen wirksame Substanzen führen nach oraler oder subkutaner Applikation zur Proliferation des Vaginalepithels und zur Verhornung der oberflächlichen Zell-Lagen.

Als Schwellenwert wird diejenige Menge eines östrobens angesehen, bei der 50 % der Tiere Stadium 3 erreichen. Die biologischen Daten sind in Tabelle 1 aufgelistet.

Tabelle 1

| Verbindungen | Allen-Doisy-Test Dosis | Zyklus-stadium | Ratten in % |
| --- | --- | --- | --- |
| 14,17ß-Ethano-14ß estra-1,3,5(10)-trien-3, 17α -diol (A) | 10 µg | 3 | > 50 |
| 14,17ß-Ehano-14ß estra-1,3,5(10), 15-tetraen-3,17α -diol (B) | 10 µg | 3 | > 50 |
| 14,17ß-Ethano-16-methyl-14ß-estra-1,3,5(10),15-tetraen-3,17α -diol (C) | 10 µg | 3 | > 50 |
| 14,17ß-Ethano-3-methoxy-14ß-estra-1,3,5(10),15-tetraen-17α -ol (D) | 10 µg | 3 | > 50 |
| 14,17ß-Ethano-3-methoxy-14ß-estra-1,3,5(10)-trien- 17α -ol (E) | 10 µg | 3 | > 50 |
| Ethinylestradiol (F) | 10 µg | 3 | < 50 |

Nach Applikation von zum Beispiel jeweils 10 µg der Verbindungen A, B, C, D und E per os wird bei über 50 % der Tiere Stadium 3 erreicht, während in der Kontrollgruppe mit 10 µg Ethinylestradiol bei keiner Ratte ein volles Stadium 3 erkennbar ist.

Es ist überraschend, daß die erfindungsgemäßen Verbindungen, die keine 17α-Ethinylgruppe enthalten, oral wirksamer sind als Ethinylestradiol. Ethinylestradiol ist nach wie vor das bei oraler Behandlung am häufigsten einbesetzte östrogen.

Die Erfindung betrifft somit Verbindungen der allgemeinen Formel I zur Verwendung bei der Behandlung von östrogenmangelerscheinungen und zur Fertilitätskontrolle bei der Frau.

Die erfindungsgmäßen Verbindungen können in der gleichen Weise wie Ethinylestradiol formuliert und eingesetzt werden. Sie werden mit den in der galenischen Pharmazie üblichen Zusätzen, Trägersubstanzen und Geschmackskorrigentien nach an sich bekannten Methoden zu den üblichen Arzneimittelformen verarbeitet. Für die orale Applikation kommen insbesondere Tabletten, Dragees, Kapseln, Pillen, Suspensionen oder Lösungen, wie zum Beispiel Sesamöl- oder Rizinusöllösungen infrage, die gegebenenfalls zusätzlich hoch ein Verdünnungsmittel, wie zum Beispiel Benzylbenzoat oder Benzylalkohol enthalten können.

Die Wirkstoffkonzentration in den pharmazeutischen Zusammensetzunben ist abhänggig von der Applikationsform und dem Anwendungsgebiet. So können zum Beispiel Kapseln oder Tabletten zu Behandlung von östrogenmangelerscheinungen 0,001 bis 0,05 mg Wirkstoff, ölige Lösungen zur intramuskulären Injektion pro 1 ml etwa 0,01 bis 0,1 mg Wirkstoff und Vaginalsalben etwa 0,1 bis 10 mg pro 100 ml Salbe enthalten. Zur Kontrazeption bei der Frau können die erfindungsgemäßen östrogene in Kombination mit Gestagenen angewandt werden. Tabletten oder Dragees zur täglichen Einnahme einer Tablette oder eines Dragees sollen vorzugsweise 0,003 bis 0,05 mg des erfindungsgemäßen östrogens und 0,05 bis 0,5 mg eines Gestagens enthalten.

Die erfindungsgmäßen Verbindungen können bei östrogenmangelerscheinunben der Frau, wie zum Beispiel Amenorrhoe, Dysmenorrhoe, Sterilität, Frigidität, Endometritis, Kolpitis und klimakterischen Beschwerden verwendet werden. Ferner können die Verbindungen als östrogene Komponente in Kombinationspräparaten mit Gestagenen zur Fertilitätskontrolle bei der Frau eingesetzt werden.

Die neuen Verbindungen der allgemeinen Formel I

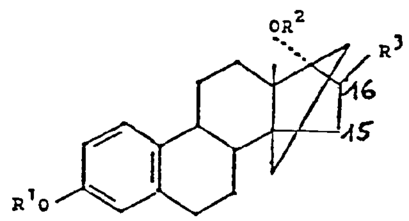

(I),

worin
$R^1$ ein Wasserstoffatom, eine Methyl- oder Acylggruppe mit 1 - 12 Kohlenstoffatomen,
$R^2$ ein Wasserstoffatom oder eine Acylgruppe mit 1 - 12 Kohlenstoffatomen,
$R^3$ ein Wasserstoffatom oder eine Methylgruppe
bedeuten, können hergestellt werden, indem man bei einer Verbindung der allgemeinen Formel I b

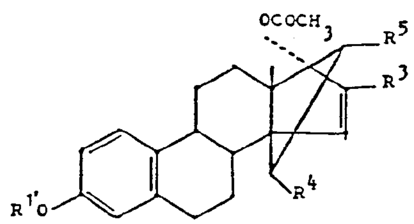

(I b),

worin
$R^{1'}$ eine Methyl- oder Acetylgruppe,
$R^3$ ein Wasserstoffatom oder eine Methylgruppe,
$R^4$ ein Wasserstoffatom und
$R^5$ eine Phenylsulfonylgruppe, wenn $R^3$ ein Wasserstoffatom,
$R^4$ ein Wasserstoffatom und
$R^5$ eine Phenylsulfonylgruppe oder
$R^4$ eine Phenylsulfonylgruppe und
$R^5$ ein Wasserstoffatom, wenn $R^3$ eine Methylgruppe
bedeuten,

die Phenylsulfonylgruppen durch Reduktion mit Amalgam oder Raney-Nickel entfernt, die 15-16-Doppelbindung hydriert, gegebenenfalls den 3-Methylether spaltet oder die 3- und/oder 17-Acetoxygruppe verseift, gegebenenfalls die 3-Hydroxygruppe partiell verestert, gegebenenfalls die 3- und 17-Hydroxyverbindungen wieder verestert und gegebenenfalls eine so erhaltene 3,17-Diacyloxy- selektiv zur 3-Hydroxy-17-acyloxyverbindung verseift.

Geeignete Reduktionsreagenzien sind Amalgam, besonders Natrium-, Kalium- und Lithiumamalgam (J. Chem. Soc., Chem. Comm. 1986, 451-453) aber auch Raney-Nickel (Synthetica Merck, Band 1, Darmstadt, 1969, Seite 439).

Die Hydrierung der 15-16-Doppelbindung erfolgt in an sich bekannter Weise, vorzugsweise in Gegenwart eines Edelmetallkatalysators auf einem inerten Träger.

Die sich ggf. anschließende Spaltung eines 3-Methylethers wird nach den üblichen Methoden der Steroidetherspaltung vorgenommen. So kann die 3-Methyletherspaltung beispielsweise mit einer Lewissäure in einem inerten Lösungsmittel in der Siedehitze durchbeführt werden. Als Lewissäuren sind beispielsweise Bortrifluoridetherat oder Diisobutylaluminiumhydrid (DIBAH) geeignet. Als Lösungsmittel kommen Benzol, Toluol, Tetrahydrofuran, Dioxan u.a. infrage.

Die Verseifung der Acyloxygruppen kann in an sich bekannter Weise erfolgen. Beispielsweise wird die Verseifung mit Basen in wäßrig-alkoholischer Lösung, wie Kaliumhydroxid in wäßrig-methanolischer Lösung, vorgenommen.

Für die sich ggf. anschließende Veresterung der phenolischen und tertiären Hydroxygruppe kommen die üblicherweise in der Steroidchemie zur Veresterung angewendeten Verfahren infrage. Beispielsweise sei die Umsetzung mit Essigsäure oder Acetanhydrid in Gegenwart starker Säuren, wie zum Beispiel Trifluoressigsäure, Perchlorsäure oder p-Toluolsulfonsäure, bei Raumtemperatur oder etwas angehobener Temperatur oder die Umsetzung mit Acetanhydrid in Gegenwart eines tertiären Amins bei etwa 20 - 80°C genannt.

Werden Pyridin und 4-Dimethylamino-pyridin als tertiäre Amine gemeinsam angewandt, kann die Veresterung vorzugsweise bei Raumtemperatur durchgeführt werden.

Die Synthesen der beiden möglichen Halbester erfolgen durch partielle Veresterung oder partielle Verseifung:

a) Ausgehend von den 3,17α-Dihydroxyverbindungen lassen sich durch selektive Veresterung der phenolischen Hydroxygruppe die 3-Acyloxy-17α-hydroxyverbindungen erhalten. Die Reaktionen werden durch Umsetzungen des entsprechenden Säureanhydrids in Gegenwart eines heterocyclischen Stickstoffaromaten, vorzugsweise Pyridin, erreicht. Als Reaktionstemperatur eignet sich der Bereich zwischen Raum- und Siedetemperatur der Reaktionsmischung.

b) Ausgehend von den 3,17α-Diacyloxyverbindungen lassen sich durch selektive Verseifung der phenolischen Acyloxygruppe die 3-Hydroxy-17α-acyloxyverbindungen erhalten. Die Synthesen erfolgen durch Umsetzungen mit einem Alkalicarbonat oder Erdalkalicarbonat, vorzugsweise Kalium oder Calciumcarbonat, in wäßrig-methanolischer Lösung. Als Reaktionstemperatur eignet sich der Bereich zwischen Raum- und Siedetemperatur der Reaktionsmischung.

Die Herstellung der Verbindungen der allgemeinen Formel Ib erfolgt durch Reaktion von 17-Acetoxy-3-methoxyestra-1,3,5(10),14,16-pentaen (Steroids 1973, 22, 107) oder 3,17-Diacetoxy-estra-1,3,5(10),14,16-pentaen (J. Org. Chem. 1972, 37, 2127) oder 17-Acetoxy-3-methoxy-16-methylestra-1,3,5(10),14,16-pentaen mit Phenylvinylsulfon. Reaktionen dieses Typs, En/Dien, werden bereits in J. Org. Chem. 1983, 48, 4976 und Steroids 1968, 11, 637 beschrieben.

Das 17-Acetoxy-3-methoxy-16-methylsestra-1,3,5(10),14,16-pentaen läßt sich aus 3-Methoxy-16-methylestra,1,3,5(10),15-tetraen-17-on (Deutsche Offenlegungsschrift 30 23 568) wie beschrieben herstellen.

Herstellung der Ausgangsverbindungen

A 14,17β-Ethano-3-methoxy-2-phenylsulfonyl-14β-estra-1,3,5(10),15-tetraen-17α-ol-acetat

Eine Mischung aus 4 g 17-Acetoxy-3-methoxy-estra1,3,5(10),14,16-pentaen (Steorids 1973, 22, 107). 6,23 g Phenylvinylsulfon und 15 ml Benzol werden 90 Stunden bei 140°C im Bombenrohr zur Reaktion gebracht. Nach dem Abkühlen wird die Reaktionsmischung an Kieselgel chromatographiert. Laufmittel: Benzol/Essigester (19:1). Es werden 5,57 g 14,17β-Ethano-3-methoxy-2-phenylsulfonyl-14β-estra-1,3,5(10),15-tetraen-17 -ol-acetat erhalten.

Schmelzpunkt: 181,5°C. $(\alpha)_D$ = +100° (Chloroform)

B 14,17β-Ethano-2-phenylsulfonyl-14β-estra-1,3,5(10),15-tetraen-3,17 -diol-diacetat

Eine Mischung aus 1,23 g 3,17-Diacetoxy-estra-1,3,5(10),14,16-pentaen (J. Org. Chem. 1972, 37, 2127), 1,77 g Phenylvinylsulfon und 4,6 ml Benzol werden 90 Stunden bei 140°C im Bombenrohr zur Reaktion gebracht. Nach dem Abkühlen wird die Reaktionsmischung an Kieselgel chromatographiert. Laufmittel: Benzol/Essigester (19:1). Es werden 1,5 g 14,17β-Ethano-2-phenylsulfonyl-14β-estra-1,3,5(10),15-tetraen-3,17 -diol-diacetat erhalten.

Schmelzpunkt: 197°C $(\alpha)_D$ = +96° (Chloroform)

C 14,17b-Ethano-3-methoxy-16-methyl-2-phenylsulfonyl-14b-estra-1,3,5(10),15-tetraen-17a-ol-acetat/ 14, 17β-Ethano-3-methoxy-16-methyl-1-phenylsulfonyl-14β-estra-1,3,5(10), 15-tetraen-17α-ol-acetat-Gemisch

20,0 g 3-Methoxy-16-methyl-estra-1,3,5(10),15 tetraen-17-on (Deutsche Offenlegungsschrift 30 23 568), 400 ml Isopropenylacetat, 80 ml Acetanhydrid und 6,0 g p-Toluolsulfonsäure werden 20 Stunden bei 100°C zur Reaktion gebracht. Nach dem Abkühlen wird die Reaktionsmischung in Eiswasser gegossen und mit festem Natriumhydrogencarbonat neutralisiert. Es wird mit Toluol extrahiert, über Natriumsulfat getrocknet und im Vakuum eingeengt. Der so erhaltene kristalline Rückstand wird an Kieselgel chromatographiert. Laufmittel: Benzol/Essigester (50:1). Es werden 19,8 g 17-Acetoxy-3-methoxy-16-methyl-estra-1,3,5(10),14,16-pentaen erhalten.

Eine Mischung aus 19,8 g 17-Acetoxy-3-methoxy-16-methyl-estra-1,3,5(10),14,16-pentaen, 10,3 g Phenylvinylsulfon und 35 ml Xylol werden 120 Stunden bei 140°C im Bombenrohr zur Reaktion gebracht. Nach dem Abkühlen wird die Reaktionsmischung an Kieselgel chromatographiert. Laufmittel: Benzol/Essigester (1:1). Es werden 22,3 g eines 1:1-Gemisches von 14,17β-Ethano-3-methoxy-16-methyl-2-phenylsulfonyl-14β-estra-1,3,5(10),15-tetraen-17d-ol-acetat und 14,17β-Ethano-3-methoxy-16-methyl-1-phenylsulfonyl-14β-estra-1,3,5(10),15-tetraen-17α-ol-acetat erhalten.

D 14,17β-Ethano-3-methoxy-14β-estra-1,3,5(10),15-tetraen-17α-ol-acetat

Es wird eine Lösung aus 2,0 g 14,17β-Ethano-3-methoxy-2-phenylsulfonyl-14β-estra-1,3,5(10),15-tetraen-17 -ol-acetat, 2,33 g wasserfreiem Dinatriumhydrogenphosphat, 40 ml Methanol und 10 ml Tetrahydrofuran hergestellt. Zu dieser Lösung gibt man unter Argon-Atmosphäre bei -20° C portionsweise 31,5 g Natriumamalgam (6 %). Es wird 3 Stunden bei -20°C gerührt. Danach wird 40 ml Wasser zugegeben. Die organische Phase wird abdekantiert und im Vakuum einbeengt. Es wird 1,42 g Rohprodukt erhalten. Dieses wird in 15 ml Acetanhydrid gelöst, mit 50 mg p-Toluolsulfonsäure versetzt und 16 Stunden bei Raumtemperatur gerührt. Danach wird in Eiswasser gegossen, mit Natriumhydrogencarbonat neutralisiert, mit Benzol extrahiert, die organische Phase getrocknet, im Vakuum vom Lösungsmittel befreit und der verbleibende Rückstand aus Methanol umkristallisiert. Es werden 1,2 g 14,17β-Ethano-3-methoxy-14β-estra-1,3,5(10),15-tetraen-17α-ol-acetat erhalten.
Schmelzpunkt: 121°C $(\alpha)_D$ = +96 (Chloroform)

E 14,17β-Ethano-14β-estra-1,3,5(10),15-tetraen-3,17α-diol

Es wird eine Lösung aus 1,05 g 14,17β-Ethano-2-phenylsulfonyl-14β-estra-1,3,5(10),15-tetraen-3,17α-diol-diacetat, 1,1 g wasserfreiem Dinatriumhydrogenphosphat, 20 ml Methanol und 5 ml Tetrahydrofuran herbestellt und auf -20°C abgekühlt. Zu dieser Lösung gibt man unter Argon-Atmosphäre portionsweise 20,0 g Natriumamalgam (6 %) und rührt 3 Stunden bei -20°C. Danach wird 20 ml Wasser zugegeben, die organische Phase abdekantiert und im Vakuum eingeengt. Es wird 0,69 g Rohprodukt erhalten. Dieses wird in 5 ml Tetrahydrofuran und 10 ml methanolischer Kalilauge (1M) gelöst und 2 Stunden bei Raumtemperatur gerührt. Die Mischung wird dann in 100 ml Eiswasser gegossen, mit verdünnter Salzsäure neutralisiert, mit Essigester extrahiert, die organische Phase getrocknet, im Vakuum eingeengt und der Rückstand aus Benzol/Essigester (1:1) umkristallisiert. Es werden 0,56 g 14,17β-Ethano-14β-estra-1,3,5(10),15-tetraen-3,17α-diol erhalten.
Schmelzpunkt: 229°C $(\alpha)_D$ = +140° (Chloroform).

F 14,17β-Ethano-14β-estra-1,3,5(10),15-tetraen-3,17α-diol-diacetat

1,25 g 14,17β-Ethano-14β-estra-1,3,5(10),15-tetraen-3,17α-diol werden in 20 ml Acetanhydrid gelöst. Zur Lösung werden 50 mg p-Toluolsulfonsäure gegeben. Es wird 16 Stunden bei Raumtemperatur gerührt, danach die Lösung in eine gesättigte Natriumhydrogencarbonatlösung gegossen, mit Benzol extrahiert, die organische Phase getrocknet und im Vakuum einbeengt. Der Rückstand wird an Kieselgel chromatographiert. Laufmittel: Benzol/Essigester (19:1). Es werden 1,5 g 14,17β-Ethano-14β-estra-1,3,5(10),15-tetraen-3,17α-diol-diacetat erhalten.
Schmelzpunkt: 103,5° C $(\alpha)_D$ = +92° (Chloroform).

G 14,17β-Ethano-3-methoxy-14β-estra-1,3,5(10),15-tetraen-17α-ol

100 ml einer methanolischen Kalilauge (1M) wird unter Rühren und Argon-Atmosphäre zu einer Lösung aus 5,3 g 14,17β-Ethano-3-methoxy-14β-estra-1,3,5(10),15-tetraen-17α-ol-acetat in 30 ml Tetrahydrofuran gegeben. Es wird 1 Stunde bei Raumtemperatur gerührt, danach in 200 ml Einswasser gegossen, mit verdünnter Salzaüre neutralisiert, mit Essigester extrahiert, die organische Phase getrocknet, im Vakuum eingeengt und der Rückstand aus Benzol/Hexan umkristallisiert. Es werden 4,4 g 14,17β-Ethano-3-methoxy-14β-estra-1,3,5(10),15-tetraen-17α-ol erhalten.
Schmelzpunkt: 152°C $(\alpha)_D$ = + 143° (Chloroform).

Beispiel 1

14,17β-Ethano-3-methoxy-14β-estra-1,3,5(10)-trien-17α-ol

Eine Lösung von 61,2 mg 14,17β-Ethano-3-methoxy-14β-estra-1,3,5(10),15-tetraen-17α-ol in 6 ml Ethylacetat wird in Gegenwart von 20 mg Palladium auf Kohle (5 %) bei Raumtemperatur unter Normaldruck hydriert. Wenn der Wasserstoffverbrauch nachläßt, wird vom Katalysator abfiltriert. Der Katalysator wird mit Ethylacetat

gewaschen und die vereinigten organischen Phasen werden im Vakuum eingedampft. Nach Filtration über eine Silicagelsäule in einem Benzol/Ethylacetat-Gemisch (9:1) erhält man 60 mg 14,17β-Ethano-3-methoxy-14β-estra-1,3,5(10)-trien-17α-ol.
Schmelzpunkt: 121°C $(\alpha)_D$ = +46° (Chloroform).

Beispiel 2

14,17β-Ethano-14β-estra-1,3,5(10)-trien-3,17α-diol

Zu einer Lösung von 129 mg 14,17β-Ethano-3-methoxy-14β-estra-1,3,5(10)-trien-17α-ol in 6 ml Toluol werden unter Rühren in einer inerten Schutzgasatmosphäre (Argon) 1,2 ml einer 1,2 molaren Lösung von Diisobutylalumimiumhydrid in Toluol gegeben. Nach 24stündigem Erhitzen unter Rückfluß wird das Reaktionsgemisch abgekühlt und mit 5 ml einer 10 %igen Salzsäurelösung verdünnt. Die wäßrige Phase wird abgetrennt und 3 x mit 25 ml Ethylacetat extrahiert. Die vereinigten organischen Phasen werden mit Kochsalzlösung gewaschen, über Natriumsulfat getrocknet und im Vakuum eingedampft. Nach chromatographie über Silicagel mit Chloroform/Methanol (19:1) erhält man 111 mg 14,17β-Ethano-14β-estra-1,3,5(10)-trien-3,17α-diol, das nach Umkristallisieren aus Chloroform/Methanol bei 241°C schmilzt. $(\alpha)_D$ = +46° (Chloroform).

Beispiel 3

14,17β-Ethano-14β-estra-1,3,5(10)-trien-3,17α-diol-diacetat

Eine Lösung von 1,40 g 14,17β-Ethano-14β-estra-1,3,5(10)-trien-3,17α-diol in 22 ml Acetanhydrid wird mit 50 mg p-Toluolsulfonsäure versetzt und 15 Stunden bei Raumtemperatur gerührt. Das Reaktionsgemisch wird mit 200 ml Wasser versetzt, das ausgefällte Produkt wird abfiltriert und in Dichlormethan aufgenommen. Die Lösung wird getrocknet und im Vakuum eingedampft. Der Rückstand wird aus Diethylether/Hexan kristallisiert.
Ausbeute: 1,42 g 14,17β-Ethano-14β-estra-1,3,5(10)-trien-3,17α-diol-diacetat.
Schmelzpunkt: 140°C $(\alpha)_D$ = +30°(Chloroform)

Beispiel 4

14,17β-Ethano-3-methoxy-14β-estra-1,3,5(10)-trien-17α-ol-acetat

Eine Lösung von 80 mg 14,17β-Ethano-3-methoxy-14β-estra-1,3,5(10)-trien-17α-ol in einem Gemisch aus 2 ml Essigsäure und 1 ml Acetanhydrid versetzt man mit 8 mg p-Toluolsulfonsäure und läßt 18 Stunden bei Raumtemperatur reagieren. Das Reaktionsprodukt wird durch Zugabe von 10 ml Wasser gefällt, abfiltriert, mit Wasser gewaschen, getrocknet und aus Pentan kristallisiert.
Ausbeute: 64 mg 14,17β-Ethano-3-methoxy-14β-estra-1,3,5(10)-trien-17α-ol-acetat.
Schmelzpunkt: 124°C $(\alpha)_D$ = +36°(Chloroform).

Beispiel 5

14,17β-Ethano-3-methoxy-14β-estra-1,3,5(10)-trien-17α-ol-propionat

Eine Lösung von 300 mg 14,17β-Ethano-3-methoxy-14β-estra-1,3,5(10)-trien-17α-ol in 3 ml Propionsäureanhydrid wird mit 10 mg p-Toluolsulfonsäure versetzt und 15 Stunden bei Raumtemperatur gerührt. Das Reaktionsgemisch wird mit 10 ml Wasser versetzt und 5 Stunden gerührt. Das ausgefällte Produkt wird abfiltriert und in Diethylether aufbenommen. Die Lösung wird getrocknet und im Vakuum eingedampft. Der Rückstand wird aus Pentan bei -20°C kristallisiert.
Ausbeute: 232 mg 14,17β-Ethano-3-methoxy-14β-estra-1,3,5(10)-trien-17α-ol-propionat.
Schmelzpunkt: 98°C $(\alpha)_D$ = +32°(Chloroform).

Beispiel 6

14,17β-Ethano-3-methoxy-14β-estra-1,3,5(10)-trien-17α-ol-hexanoat

Die Veresterung von 14,17β-Ethano-3-methoxy-14β-estra-1,3,5(10)-trien-17α-ol mit Caprosäureanhydrid erfolgt analog Beispiel 5. Es wird 14,17β-Ethano-3-methoxy-14β-estra-1,3,5(10)-trien-17α-ol-hexanoat erhalten.

Beispiel 7

14,17β-Ethano-14β-estra-1,3,5(10)-trien-3,17α-diol-dipropionat

Eine Lösung von 200 mg 14,17β-Ethano-14β-estra-1,3,5(10)-trien-3,17α-diol in 5 ml Propionsäureanhydrid wird mit 10 mg p-Toluolsulfonsäure versetzt und 15 Stunden bei Raumtemperatur gerührt. Das Reaktionsgemisch wird mit 10 ml Wasser versetzt und 1 Stunde gerührt. Das ausgefällte Produkt wird abfiltriert und in Diethylether aufgenommen. Die Lösung wird getrocknet und im Vakuum eingedampft. Der Rückstand wird aus Diethlether/Hexan kristallisiert.
Ausbeute: 222 mg 14,17β-Ethano-14β-estra-1,3,5(10)-trien-3,17α-diol-dipropionat.
Schmelzpunkt: 137°C $(\alpha)_D$ = +27° (Chloroform).

Beispiel 8

14,17β-Ethano-14β-estra-1,3,5(10)-trien-3,17α-diol dibutyrat

Die Veresterung von 14,17β-Ethano-14β-estra-1,3,5(10)-trien-3,17α-diol mit Buttersäureanhydrid erfolgt analog Beispiel 7. Es wird 14,17β-Ethano-14β-estra-1,3,5(10)-trien-3,17α-diol-dibutyrat erhalten.

Beispiel 9

14,17β-Ethano-14β-estra-1,3,5(10)-trien-3,17α-diol-diisobutyrat

Die Veresterung von 14,17β-Ethano-14β-estra-1,3,5(10)-trien-3,17α-diol mit Isobuttersäureanhydrid erfolgt analog Beispiel 7. Es wird 14,17β-Ethano-14β-estra-1,3,5(10)-trien-3,17α-diol-diisobutyrat erhalten.

Beispiel 10

14,17β-Ethano-14β-estra-1,3,5(10)-trien-3,17α-diol-dihexanoat

Die Veresterung von 14,17β-Ethano-14β-estra-1,3,5(10)-trien-3,17α-diol mit Capronsäureanhydrid erfolgt analog Beispiel 7. Es wird 14,17β-Ethano-14β-estra-1,3,5(10)-trien-3,17α-diol-hexanoat erhalten.

Beispiel 11

14,17β-Ethano-14β-estra-1,3,5(10)-trien-3,17α-diol-diundecanoat

Eine Lösung von 250 mg 14,17β-Ethano-14β-estra-1,3,5(10)-trien-3,17α-diol und 30 mg Dimethylaminopyridin in 4 ml Pyridin und 2 ml Undecansäureanhydrid wird 8 Stunden auf 60°C erhitzt. Nach dem Abkühlen wird das Reaktionsgemisch mit 15 ml Eiswasser versetzt, 24 Stunden bei Raumtemperatur gerührt und mit pentan extrahiert. Der Extrakt wird getrocknet, im Vakuum eingeengt und der Rückstand an Kieselgel chromatographiert.
Ausbeute: 273 mg 14,17β-Ethano-14β-estra-1,3,5(10)-trien-3,17α-diol-diundecanoat als zähes Öl. $(\alpha)_D$ = +17° (Chloroform).

Beispiel 12

14,17β-Ethano-14β-estra-1,3,5(10)-trien-3,17α-diol-dibenzoat

Die Veresterung von 14,17β-Ethano-14β-estra-1,3,5(10)-trien-3,17α-diol mit Benzoesäureanhydrid erfolgt analog Beispiel 7. Es wird 14,17β-Ethano-14β-estra-1,3,5(10)-trien-3,17α-diol-dibenzoat erhalten.

Beispiel 13

14,17β-Ethano-14β-estra-1,3,5(10)-trien-3,17α-diol-17-acetat

Eine Lösung von 500 mg 14,17β-Ethano-14β-estra-1,3,5(10)-trien-3,17α-diol-3,17-diacetat in einem Gemisch aus 12 ml Methanol und 2,5 ml Wasser wird mit 500 mg Calciumcarbonat versetzt und 50 Stunden zum Sieden erhitzt. Das Reaktionsgemisch wird filtriert, das filtrat im Vakuum zur Trockne gedampft und der Rückstand aus Dichlormethan/Diisopropylether kristallisiert.
Ausbeute: 174 mg 14,17β-Ethano-14β-estra-1,3,5(10)-trien-3,17-diol-17-acetat.
Schmelzpunkt: 250°C $(\alpha)_D$ = +32° (Chloroform)

Beispiel 14

14,17β-Ethano-14β-estra-1,3,5(10)-trien-3,17α-diol-17-propionat

Eine Lösung von 120 mg 14,17β-Ethano-14β-estra-1,3,5(10)-trien-3,17α-diol-3,17-dipropionat in einem Gemisch aus 6 ml Methanol und 1 ml Wasser wird mit 200 mg Calciumcarbonat versetzt und 60 Stunden zum

Sieden erhitzt. Das Reaktionsgemisch wird filtriert, das Filtrat im Vakuum zur Trockne gedampft und der Rückstand aus Dichlormethan/Diisopropylether/-Hexan kristallisiert.
Ausbeute: 76 mg 14,17β-Ethano-14β-estra-1,3,5(10)-trien-3,17α-diol-17-propionat.
Schmelzpunkt: 237°C $(\alpha)_D$ = +33° (Chloroform).

Beispiel 15

14,17β-Ethano-14β-estra-1,3,5(10)-trien-3,17α-diol-3-acetat
Eine Lösung von 270 mg 14,17β-Ethano-14β-estra-1,3,5(10)-trien-3,17α-diol in einem Gemisch aus 5 ml Pyridin und 2,5 ml Acetanhydrid wird 3 Stunden bei Raumtemperatur gerührt. Das Reaktionsgemisch wird mit 10 ml Eiswasser versetzt, das ausgefällte Produkt wird filtriert und in Dichlormethan gelöst. Die Lösung wird getrocknet, im Vakuum eingedampft und das Rohprodukt aus Dichlormethan/Diisopropylether kristallisiert.
Ausbeute: 142 mg 14,17β-Ethano-14β-estra-1,3,5(10)-trien-3,17α-diol-3-acetat.
Schmelzpunkt: 162°C $(\alpha)_D$ = +40,5° (Chloroform).

Beispiel 16

14,17β-Ethano-14β-estra-1,3,5(10)-trien-3,17α-diol-3-propionat
Eine Lösung von 50 mg 14,17β-Ethano-14β-estra-1,3,5(10)-trien-3,17α-diol in einem Gemisch aus 1 ml Pyridin und 0,5 ml Propionsäureanhydrid wird 3 Stunden bei Raumtemperatur stehen gelassen. Das Reaktiongemisch wird mit 10 ml Eiswasser versetzt und 1 Stunde gerührt. Das ausgefällte Produkt wird filtriert und in Dichlormethan gelöst, die Lösung wird getrocknet, im Vakuum eingedampft und das Rohprodukt aus Diethylether/Hexan kristalliert.
Ausbeute: 42 mg 14,17β-Ethano-14β-estra-1,3,5(10)-trien-3,17α-diol-3-propionat.
Schmelzpunkt: 146°C. $(\alpha)_D$ = +41° (Chloroform).

Beispiel 17

14,17β-Ethano-3-methoxy-14β-estra-1,3,5(10)-trien-17α-ol-butyrat
Eine Lösung von 200 mg 14,17β-Ethano-3-methoxy-14β-estra-1,3,5(10)-trien-17α-ol in 2 ml Pyridin und 1 ml Buttersäureanhydrid wird mit 20 mg Dimethylaminopyridin versetzt und 24 Stunden bei Raumtemperatur stehen gelassen. Das Reaktionsgemisch wird mit 10 ml Eiswasser versetzt und 3 Stunden gerührt. Das ausgefällte Produkt wird filtriert, mit Wasser gewaschen, an der Luft getrocknet und bei -20°C aus Pentan kristallisiert.
Ausbeute: 119 mg 14,17β-Ethano-3-methoxy-14β-estra-1,3,5(10)-trien-17α-ol-butyrat.
Schmelzpunkt: 86°C $(\alpha)_D$ = +32° (Chloroform)

Beispiel 18

14,17β-Ethano-3-methoxy-14β-estra-1,3,5(10)-trien-17α-ol-decanoat
Eine Lösung von 200 mg 14,17β-Ethano-3-methoxy-14β-estra-1,3,5(10)-trien-17α-ol in 3 ml Pyridin und 1,5 ml Decansäureanhydrid wird mit 20 mg Dimethylaminopyridin versetzt und 6 Stunden auf 60°C erhitzt. Nach dem Abkühlen wird zum Reaktionsgemisch 10 ml Eiswasser hinzugefügt, 24 Stunden bei Raumtemperatur gerührt und mit Pentan extrahiert. Der Extrakt wird getrocknet, im Vakuum eingeengt und der Rückstand an Kieselgel chromatographiert. Man erhält 264 mg öliges Produkt, das bei -70°C aus Pentan 68 mg kristallines 14,17β-Ethano-3-methoxy-14β-estra-1,3,5(10)-trien-17α-ol-decanoat ergibt.
Schmelzpunkt: 29°C $(\alpha)_D$ = +24° (Chloroform).

Beispiel 19

0,003 g 14,17β-Ethano-14β-estra-1,3,5(10)-trien-3,17α-diol und 209,997 g Lactose werden homogen gemischt und jeweils 210 mg dieser Mischung in Hart-gelatine-Steckkapseln der Größe 3 gefüllt.

Beispiel 20

Tabletten können in ühlicher Weise aus folgenden Bestandteilen hergestellt werden:

| | |
|---|---|
| 0,025 mg | 14,17ß-Ethano-14ß-estra-1,3,5(10)-trien-3,17α-diol |
| 0,150 mg | 17α-Ethinyl-17ß-hydroxy-18-methyl-4-estren-3-on (Levonorgestrel) |
| 55,225 mg | Lactose |
| 24,000 mg | mikrokristalline Cellulose |
| 0,600 mg | Magnesiumstearat |
| --------- | |
| 80,000 mg | Gesamtgewicht der Tablette |

## Patentansprüche

1. östrogen wirksames Arzneimittel, enthaltend eine Verbindung der allgemeinen Formel I

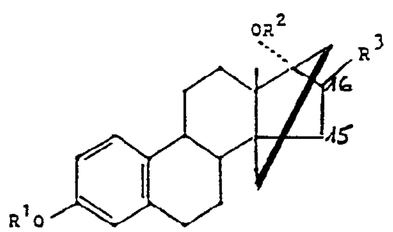

(I),

worin
$R^1$ ein Wasserstoffatom, eine Methyl- oder eine Acylgruppe mit 1 - 12 Kohlenstoffatomen,
$R^2$ ein Wasserstoffatom oder eine Acylgruppe mit 1 - 12 Kohlenstoffatomen,
$R^3$ ein Wasserstoffatom oder eine Methylgruppe
bedeuten, sowie die in der galenischen Pharmazie üblichen Zusätze.

2. 14,17β-Ethano-14β-estratriene der allgemeinen Formel I

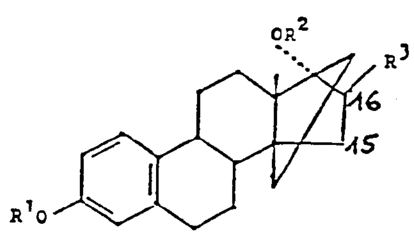

(I ),

worin
$R^1$ ein Wasserstoffatom, eine Methyl- oder eine Acylgruppe mit 1 - 12 Kohlenstoffatomen,
$R^2$ ein Wasserstoffatom oder eine Acylgruppe mit 1 - 12 Kohlenstoffatomen,
$R^3$ ein Wasserstoffatom oder eine Methylgruppe
bedeuten.

3. Verbindungen gemäß Anspruch 2
14,17β-Ethano-14β-estra-1,3,5(10)-trien-3,17α-diol,
14,17β-Ethano-3-methoxy-14β-estra-1,3,5(10)-trien-17α-ol,
14,17β-Ethano-3-methoxy-14β-estra-1,3,5(10)-trien-17α-ol-butyrat,
14,17β-Ethano-14β-estra-1,3,5(10),trien-3,17α-diol-diacetat,
14,17β-Ethano-3-methoxy-14β-estra-1,3,5(10)-trien-17α-ol-acetat,
14,17β-Ethano-14β-estra-1,3,5(10)-trien-3,17α-diol-3-acetat,

14,17β-Ethano-14β-estra-1,3,5(10)-trien-3,17α-diol-17-acetat,
14,17β-Ethano-14β-estra-1,3,5(10)-trien-3,17α-diol-3-propionat,
14,17β-Ethano-14β-estra-1,3,5(10)-trien-3,17α-diol-17-propionat,
14,17β-Ethano-3-methoxy-14β-estra-1,3,5(10)-trien-17α-ol-propionat,
14,17β-Ethano-3-methoxy-14β-estra-1,3,5(10)-trien-17α-ol-hexanoat,
14,17β-Ethano-3-methoxy-14β-estra-1,3,5(10)-trien-17β-ol-decanoat,
14,17β-Ethano-14β-estra-1,3,5(10)-trien-3,17α-diol-dipropionat
14,17β-Ethano-14β-estra-1,3,5(10)-trien-3,17α-diol-dibutyrat
14,17β-Ethano-14β-estra-1,3,5(10)-trien-3,17α-diol-diisobutyrat,
14,17β-Ethano-14β-estra-1,3,5(10)-trien-3,17α-diol-dihexanoat,
14,17β-Ethano-14β-estra-1,3,5(10)-trien-3,17α-diol-diundecanoat
und
14,17β-Ethano-14β-estra-1,3,5(10)-trien-3,17α-diol-dibenzoat.

4. Verfahren zur Herstellung von Verbindungen der allgemeinen Formel I

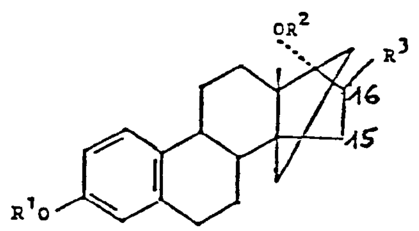

(I),

worin
$R^1$ ein Wasserstoffatom, eine Methyl- oder eine Acylgruppe mit 1 - 12 Kohlenstoffatomen,
$R^2$ ein Wasserstoffatom oder eine Acylgruppe mit 1 - 12 Kohlenstoffatomen,
$R^3$ ein Wasserstoffatom oder eine Methylgruppe
ist,
dadurch gekennzeichnet, daß
man bei Verbindung der allgemeinen Formel Ib

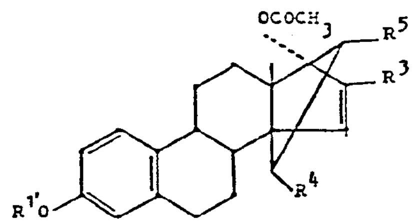

(I b),

worin
$R^{1'}$ eine Methyl- oder Acetylgruppe,
$R^3$ ein Wasserstoffatom oder eine Methylgruppe
$R^4$ ein Wasserstoffatom und
$R^5$ eine Phenylsulfonylgruppe, wenn $R^3$ ein Wasserstoffatom,
$R^4$ ein Wasserstoffatom und
$R^5$ eine Phenylsulfonylgruppe oder
$R^4$ eine Phenylsulfonylgruppe und
$R^5$ ein Wasserstoffatom, wenn $R^3$ eine Methylgruppe
bedeuten,

Die Phenylsulfonylgruppe durch Reduktion mit Amalgam oder Raney-Nickel entfernt, die 15-16-Doppelbindung hydriert, ggf. den 3-Methylether spaltet oder die 3- und/oder 17-Acetroxygruppe verseift, ggf. die 3-Hydroxygruppe partiell verestert, ggf. die 3- und 17-Hydroxyverbindungen wieder verestert und ggf. eine so erhaltene 3,47-Diacyloxy- selektiv zur 3-Hydroxy-17-acyloxyverbindung verseift.

5. Verfahren zur Herstellung von östrogen wirksamen Arzneimitteln, enthaltend eine Verbindung der allgemeinen Formel I,
dadurch gekennzeichnet, daß

man den Wirkstoff mit den in der Galenik üblichen Zusätzen mischt und in eine für die orale oder parenterale Applikation geeignete Form bringt.

## Claims

1. Medicament having oestrogenic activity, comprising a compound of the general formula I

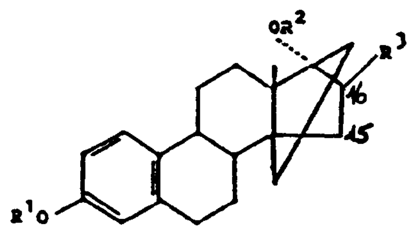

(I),

wherein
$R^1$ represents a hydrogen atom, a methyl group or an acyl group having from 1 to 12 carbon atoms,
$R^2$ represents a hydrogen atom or an acyl group having from 1 to 12 carbon atoms, and
$R^3$ represents a hydrogen atom or a methyl group,
together with the additives customary in galenic pharmacy.

2. 14,17β-ethano-14β-oestratrienes of the general formula I

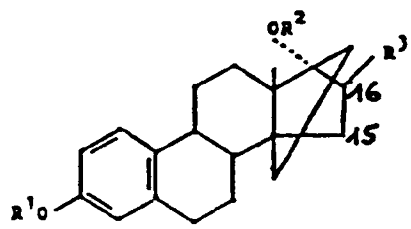

(I),

wherein
$R^1$ represents a hydrogen atom, a methyl group or an acyl group having from 1 to 12 carbon atoms,
$R^2$ represents a hydrogen atom or an acyl group having from 1 to 12 carbon atoms, and
$R^3$ represents a hydrogen atom or a methyl group.

3. Compounds according to claim 2
14,17β-ethano-14β-oestra-1,3,5(10)-triene-3,17α-diol,
14,17β-ethano-3-methoxy-14β-oestra-1,3,5(10)-trien-17α-ol,
14,17β-ethano-3-methoxy-14β-oestra-1,3,5(10)-trien-17α-ol butyrate,
14,17β-ethano-14β-oestra-1,3,5(10)-triene-3,17α-diol diacetate,
14,17β-ethano-3-methoxy-14β-oestra-1,3,5(10)-trien-17α-ol acetate,
14,17β-ethano-14β-oestra-1,3,5(10)-triene-3,17α-diol 3-acetate,
14,17β-ethano-14β-oestra-1,3,5(10)-triene-3,17α-diol 17-acetate,
14,17β-ethano-14β-oestra-1,3,5(10)-triene-3,17α-diol 3-propionate,
14,17β-ethano-14β-oestra-1,3,5(10)-triene-3,17α-diol 17-propionate,
14,17β-ethano-3-methoxy-14β-oestra-1,3,5(10)-trien-17α-ol propionate,
14,17β-ethano-3-methoxy-14β-oestra-1,3,5(10)-trien-17α-ol hexanoate,
14,17β-ethano-3-methoxy-14β-oestra-1,3,5(10)-trien-17α-ol decanoate,
14,17β-ethano-14β-oestra-1,3,5(10)-triene-3,17α-diol dipropionate,
14,17β-ethano-14β-oestra-1,3,5(10)-triene-3,17α-diol dibutyrate,
14,17β-ethano-14β-oestra-1,3,5(10)-triene-3,17α-diol diisobutyrate,
14,17β-ethano-14β-oestra-1,3,5(10)-triene-3,17α-diol dihexanoate,
14,17β-ethano-14β-oestra-1,3,5(10)-triene-3,17α-diol diundecanoate, and
14,17β-ethano-14β-oestra-1,3,5(10)-triene-3,17α-diol dibenzoate.

4. Process for the preparation of compounds of the general formula I

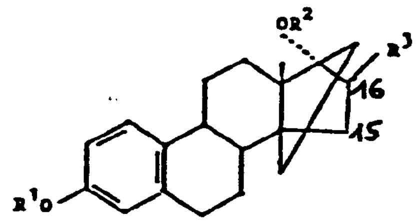

(I)

wherein
$R^1$ is a hydrogen atom, a methyl group or an acyl group having from 1 to 12 carbon atoms,
$R^2$ is a hydrogen atom or an acyl group having from 1 to 12 carbon atoms, and
$R^3$ is a hydrogen atom or a methyl group,
characterised in that
in a compound of the general formula Ib

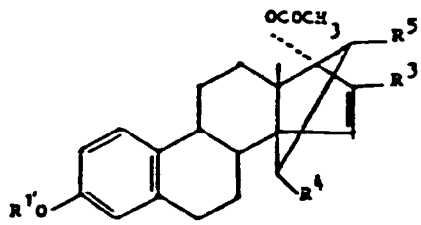

(Ib),

wherein
$R^1$ represents a methyl group or an acetyl group,
$R^3$ represents a hydrogen atom or a methyl group,
$R^4$ represents a hydrogen atom and
$R^5$ represents a phenylsulphonyl group when $R^3$ represents a hydrogen atom,
$R^4$ represents a hydrogen atom and
$R^5$ represents a phenylsulphonyl group or
$R^4$ represents a phenylsulphonyl group and
$R^5$ represents a hydrogen atom when $R^3$ represents a methyl group,
the phenylsulphonyl groups are removed by reduction with amalgam or Raney nickel, the 15-16 double bond is hydrogenated, where appropriate the 3-methyl ether is cleaved or the 3- and/or 17-acetoxy group is saponified, where appropriate the 3-hydroxy group is partially esterified, where appropriate the 3- and 17-hydroxy compounds are esterified again and where appropriate a 3,17-diacyloxy compound so obtained is saponified selectively to form the 3-hydroxy-17-acyloxy compound.

5. Process for the preparation of medicaments having oestrogenic activity and comprising a compound of the general formula I,
characterised in that
the active ingredient is mixed with the additives customary in galenics and is brought into a form suitable for oral or parenteral administration.

## Revendications

1. Médicament doué d'une activé d'oestrogène qui contient un composé répondant à la formule générale I :

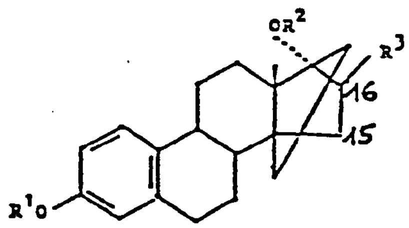

dans laquelle
R¹ représente un atome d'hydrogène, un radical méthyle ou un radical acyle contenant de 1 à 12 atomes de carbone,
R² représente un atome d'hydrogène ou un radical acyle contenant de 1 à 12 atomes de carbone, et
R³ représente un atome d'hydrogène ou un radical méthyle,
ainsi que des additifs pris parmi ceux qu'on utilise habituellement en pharmacie.

2. 14,17β-Ethano-14β-estratriènes répondant à la formule générale I :

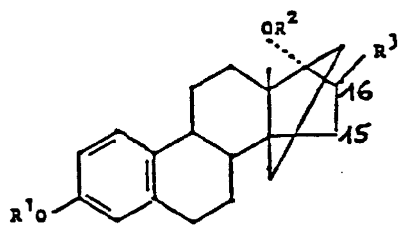

dans laquelle
R¹ représente un atome d'hydrogène, un radical méthyle ou un radical acyle contenant de 1 à 12 atomes de carbone,
R² représente un atome d'hydrogène ou un radical acyle contenant de 1 à 12 atomes de cabone et
R³ représente un atome d'hydrogène ou un radical méthyle.

3. Composés selon la revendication 2, en l'espèce :
14,17β-éthano-14β-estra-1,3,5(10)-triène-3,17α-diol,
14,17β-éthano-3-méthoxy-14β-estra-1,3,5(10)-triène-17α-ol,
butyrate du 14,17β-éthano-3-méthoxy-14β-estra-1,3,5(10)-triène-17α-ol,
diacétate du 14,17β-éthano-14β-estra-1,3,5(10)-triène-3,17α-diol,
acétate du 14,17β-éthano-3-méthoxy-14β-estra-1,3,5(10)-triène-17α-ol,
3-acétate du 14,17β-éthano-14β-estra-1,3,5(10)-triène-3,17α-diol,
17-acétate du 14,17β-éthano-14β-estra-1,3,5(10)-triène-3,17α-diol,
3-propionate du 14,17β-éthano-14β-estra-1,3,5(10)-triène-3,17α-diol,
17-propionate du 14,17β-éthano-14β-estra-1,3,5(10)-triène-3,17α-diol,
propionate du 14,17β-éthano-3-méthoxy-14β-estra-1,3,5(10)-triène-17α-ol,
hexanoate du 14,17β-éthano-3-méthoxy-14β-estra-1,3,5(10)-triène-17α-ol,
décanoate du 14,17β-éthano-3-méthoxy-14β-estra-1,3,5(10)-triène-17α-ol,
dipropionate du 14,17β-éthano-14β-estra-1,3,5(10)-triène-3,17α-diol,
dibutyrate du 14,17β-éthano-14β-estra-1,3,5(10)-triène-3,17α-diol,
diisobutyrate du 14,17β-éthano-14β-estra-1,3,5(10)-triène-3,17α-diol,
dihexanoate du 14,17β-éthano-14β-estra-1,3,5(10)-triène-3,17α-diol,
diundécanoate du 14,17β-éthano-14β-estra-1,3,5(10)-triène-3,17α-diol,
dibenzoate du 14,17β-éthano-14β-estra-1,3,5(10)-triène-3,17a-diol.

4. Procédé pour préparer des composés répondant à la formule générale I

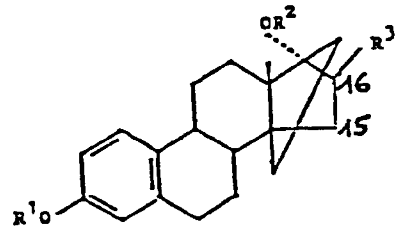

dans laquelle
$R^1$ représente un atome d'hydrogène, un radical méthyle ou un radical acyle contenant de 1 à 12 atomes de carbone,
$R^2$ représente un atome d'hydrogène ou un radical acyle contenant de 1 à 12 atome de cabone et
$R^3$ représente un atome d'hydrogène ou un radical méthyle.
procédé caractérisé en ce que, dans un composé répondant à la formule générale Ib :

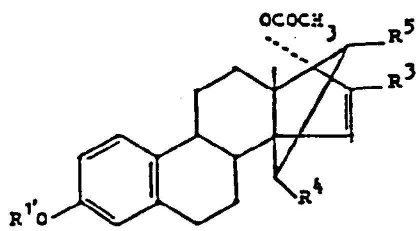

dans laquelle
$R^{1'}$ représente un radical méthyle ou acétyle,
$R^3$ représente un atome d'hydrogène ou un radical méthyle,
- dans le cas où $R^3$ représente un atome d'hydrogène :
$R^4$ représente un atome d'hydrogène et
$R^5$ un radical phénylsulfonyle,
- dans le cas où $R^3$ représente un radical méthyle :
$R^4$ représente un atome d'hydrogène et
$R^5$ un radical phénylsulfonyle, ou
$R^4$ représente un radical phénylsulfonyle et
$R^5$ un atome d'hydrogène,
on élimine les radicaux phénylsulfonyles par réduction au moyen d'un amalgame ou de nickel de Raney, on hydrogène la double liaison 15,16, éventuellement on coupe l'éther méthylique en 3 ou on saponifie le radical acétoxy en 3 et/ou en 17, éventuellement on estérifie partiellement le radical hydroxy en 3, éventuellement on estérifie à nouveau les composés porteurs d'un hydroxy en 3 et en 17 et éventuellement on saponifie sélectivement un composé 3,17-diacyloxy ainsi obtenu pour le convertir en le composé 3-hydroxy-17-acyloxy.

5. Procédé pour préparer des médicaments doués d'une activité oestrogène qui contiennent un composé de formule générale I, procédé caractérisé en ce qu'on mélange la substance active avec des additifs usuels pris parmi ceux qu'on utilise couramment en pharmacie, et on met le mélange sous une forme appropriée pour l'application orale ou parentérale.